**Europäisches Patentamt**

**European Patent Office**

**Office européen des brevets**

(19)

(11) Publication number: **0 251 654**
**A2**

# EUROPEAN PATENT APPLICATION

(12)

(21) Application number: **87305573.5**

(22) Date of filing: **23.06.87**

(51) Int. Cl.⁴: **C12N 15/00** , **A01H 1/00** ,
**C12N 5/00**

(30) Priority: **23.06.86 US 877261**

(43) Date of publication of application:
**07.01.88 Bulletin 88/01**

(84) Designated Contracting States:
**AT BE CH DE ES FR GB GR IT LI LU NL SE**

(71) Applicant: **BIOTECHNICA INTERNATIONAL, INC.**
**85 Bolton Street**
**Cambridge Massachusetts02140(US)**

(72) Inventor: **Cannon, Maura C.**
**40 Prospect Hill Road**
**Lexington Massachusetts(US)**
Inventor: **Cannon, Frank C.**
**40 Prospect Hill Road**
**Lexington Massachusetts(US)**

(74) Representative: **Deans, Michael John Percy et al**
**Lloyd Wise, Tregear & CO. Norman House**
**105-109 Strand**
**London WC2R OAE(GB)**

(54) **Transformation of chloroplasts.**

(57) A plant having plastids, particularly chloroplasts, genomes contain heterologous DNA.

FIG Ia

FIG Ib

## TRANSFORMATION OF CHLOROPLASTS

This invention relates to genetic engineering of plants.

By the term "heterologous DNA", we mean DNA which is a non-plant gene, a modified gene, a gene from a different plant strain or species, or a homologous gene from a different location in the plant nuclear, chloroplast, or mitochondrial genome. By the term "genome", we mean all DNA within a cell or organelle, i.e., chromosomal and other autonomously replicating DNAs.

Chloroplasts are the site of photosynthesis in plants. These organelles are composed of an outer membrane and an interior network of stacks of sac-like membranous structures called thylakoids. Using the energy of light, the photosynthetic machinery (enzymes and other proteins) of the plant converts carbon dioxide and water to carbohydrate and oxygen. These photosynthetic enzymes and proteins are found either free in the thylakoid matrix (stroma) or embedded in the thylakoid membrane, which provides a sheltered and highly sterically structured environment for chloroplast functions which require such conditions. Chloroplasts possess their own circular DNA and protein synthesis machinery.

Chloroplasts belong to a class of subcellular organelles called "plastids", which originate from undifferentiated subcellular organelles called "proplastids". Chloroplasts are the major type of plastid. Other plastid types include chromoplasts (which give color to non-green parts of plants) and leucoplasts (whose function is not fully known). See standard texts such as "Biology of Plants", P.H. Raven & R.F. Evert; H. Curtis, Worth Publishers, Inc., New York, 1981, pages 22-23.

Boissel et al., Experientia (1983) 39:658 report cloning and sequencing autonomously replicating DNA fragments from the chloroplast genome of Chlamydomonas reinhardii. Inserted into a pBR322 plasmid derivative, the fragments were observed to replicate in yeast.

Oyama, Biotech. Newswatch, p7, 10/3/83 reports the construction of a vector composed of a pBR322-derived E. coli Promoter, a pKC7 kanamycin resistance marker, and a fragment of liverwort chloroplast DNA containing two origins of replication. It is suggested that this and similar constructs may be used as autonomously replicating transformation vehicles in chloroplasts.

Grinter European Patent Application No. 83300542.4 describes cloning vectors for the integration of foreign DNA into the chromosomes of bacteria. The vectors contain DNA derived from the transposon, Tn7, carrying a gene for antibiotic resistance and a desired foreign gene.

Arntzen et al., The Fourteenth Lynen Lecture, Academic Press, Inc., New York (1983) pp 273-294 describes the isolation, cloning, and sequencing of the gene for triazine herbicide resistance. The gene, a modified version of "photogene 32", resides in the chloroplast genome of resistant plants. The product of the normal version of photogene 32 is the receptor for the herbicide, and is an essential component of the photosynthetic machinery of the chloroplast. The normal photogene 32 product accepts triazine as a substrate and becomes inactivated. The product of the modified version of photogene 32, found in triazine-resistant plants, does not recognize triazines as substrates, and can function normally even in the presence of high concentrations of the herbicide. It is suggested that chloroplasts could be transformed to triazine resistance by introduction of the modified, resistance gene. The transformed triazine-resistant chloroplasts could be introduced into triazine-sensitive plant cells, and confer triazine-resistance.

We describe below plants having plastids, preferably chloroplasts, containing heterologous DNA; vectors capable of effecting the integration of a portion of itself into the genome of a plant plastid; and plant cells and plants derived therefrom.

According to a first aspect of the present invention, there is provided a plant having plastids in which the genomes of said plastids contain heterologous DNA.

The invention provides, in a second and alternative aspect thereof, a vector capable of effecting the integration of a portion of itself into the genome of a plastid, said vector containing a transposon including a gene encoding a selectable marker protein and having insertion sequences on either side of said gene encoding said selectable marker portion, said vector further comprising, on either side of said insertion sequences, a first and second DNA region which together comprise a DNA region homologous with a DNA region of said genome of said plastid. The invention extends to the product of use of such vectors, such as a plastid in which the genome thereof has integrated into it the aforementioned integrating portion of the vector, plant cells containing such plastids, and plants comprising a plurality of such cells. The invention also extends to methods using such vectors (and to the direct products of such methods) such as a method of producing a plant whose cells express a desired gene, the method comprising isolating plastids from a plant; transforming said plastids with an aforementioned vector; inserting said transformed plastids into plant cells; and regenerating a plant from said plant cells.

In a yet further aspect of the present invention, there is provided a plastid which is transformed with heterologous DNA.

In preferred embodiments, the integrated portion of the vector includes a gene for a selectable marker protein and a desired DNA sequence, or site for the insertion thereof. Preferably the vector also includes a transposon having insertion sequences on either side of the gene for the selectable marker protein, i.e., the marker protein gene is in the transposon "loop." Preferably, on either side of the insertion sequences there are DNA regions homologous with plastid genome regions, to direct the vector to a desired (preferably transcriptionally silent) region.

The invention permits the production in plant cells of proteins which confer a beneficial trait on the plant, e.g., herbicide resistance, and which are advantageously produced in the sheltered environment of the plastid, e.g., chloroplast. In addition, plastid genome integration provides for stability of the inserted DNA over multiple cell divisions.

Other features and advantages of the invention will be apparent from the following description of the preferred embodiments thereof.

We now describe preferred embodiments of the invention, after briefly describing the drawings.

Figs. la and b are diagrammatic representations of the structure and construction of an embodiment of vector in accordance with (a) or without (b) a herbicide resistance gene.

Fig. 2 is a diagrammatic representation of the genomes of the chloroplasts of maize, spinach, peas, and tobacco, showing the intergenic regions of each. TS represents a transcriptional start site; atpB encodes for Beta-ATPase; and rbcL encodes the large subunit of ribulose biphosphate carboxylase. The intergenic region is shown between the dotted lines.

Figs. 3 and 4 are diagrammatic representations of vectors containing cloned DNA including portions of two corn chloroplast genes.

Fig. 5 is a diagrammatic representation of a subclone of the vectors of Figs. 3 and 4.

Fig. 6 is an illustration of the sites of insertion of the transposon Tn5 into the intergenic regions in the vector of Fig. 5.

Fig. 7 is a diagrammatic representation of an embodiment of vector in accordance with the invention containing cloned tobacco chloroplast DNA.

Figs. 8-14 are diagrammatic representations of the structure and construction of intermediates used in the construction of the vector of figure 7.

Fig. l5 is a diagrammatic representation of an embodiment of broad-host range vector in accordance with the invention.

Figs. 16-23 are diagrammatic representations of embodiments of vectors in accordance with the invention, or intermediates used in the construction thereof, which do not contain chloroplast DNA.

The following discussion is in terms of chloroplasts, but is applicable to other plastids as well.

## Vector Components

As mentioned above, vectors in accordance with the invention can contain several DNA regions and sites, now discussed in greater detail.

## Integrating DNA

Integration of the vector carrying the desired heterologous gene into the genome of the chloroplast is facilitated if the vector either contains DNA homologous with a region of chloroplast DNA, or includes a transposon, or both, although integration can occur with neither at a certain, albeit lower, frequency. Integration via homologous DNA occurs via classical homologous crossing over, while integration via transposon DNA does not involve the classical crossing over, and does not require regions of extensive DNA homology. Transposons are DNA segments, found in both prokaryotes and eukaryotes, which assume loop and stem configurations and contain inverted repeat insertion sequences which can effect the insertion of the DNA in the transposon loop into any of a number of locations on the chloroplast genome. Thus, random chromosomal site integration using transposon DNA will result in the integration of the desired DNA in the chromosome at some sites which are advantageous, and other sites which deleteriously disrupt essential chloroplast genes. Therefore, it is advantageous to direct the integration of the transposon containing the desired DNA to a site in the chloroplast genome where the transcriptional integrity of the crucial native genes will be preserved. To achieve this, the transposon, prior to insertion, is inserted into a fragment of chloroplast DNA (cpDNA) such that the insertion occurs in a region known to be transcriptionally silent. A suitable such region is one between two known promoters of chloroplast genes, e.g., the promoters for the genes for the chloroplast "large subunit of ribulose bisphosphate carboxylase" and for beta-ATPase. The fragment of chloroplast DNA in the transposon is, at least in part, homologous with a sequence on the host chloroplast DNA. When the vector containing the fragment of cpDNA and the inserted transposon is introduced into a chloroplast, the fragment associates with its region of homology in the genome of the chloroplast. This association allows the site-directed integration of the transposon by either reciprocal recombination or, preferably, homogenitization (i.e., gene conversion) of the transposon. Thus insertion can be directed to a locus, preferably a transcriptionally silent region, at which gene inactivation and subsequent loss of function are not caused.

Origin of Replication

Although integration is preferred, for the reasons cited above, other vectors in accordance with the invention are maintained in chloroplasts by virtue of origins of replication enabling autonomous DNA replication. Suitable origins include those from P-type and Q-type incompatibility plasmids (wide-host range plasmids of Gram-negative bacteria), such as pSUPI04.

Selectable Marker

Because transformation of plant cells with vectors containing foreign genes is a relatively rare event, vectors in accordance with the invention preferably contain a DNA region which encodes a selectable marker protein for identificaton of transformants. This marker protein can be any protein which can be expressed in plant cells and which enables the phenotypic identification of plant cells which express the protein. Preferred marker proteins are proteins which provide resistance to one or more herbicides or antibiotics; currently most preferred is the protein aminoglycoside phosphotransferase (APHII), which inactivates antibiotics such as kanamycin, neomycin, and G4l8; transformants are those plant cells able to grow in the presence of antibiotic. The desired heterologous gene can also double as the marker gene, e.g., in the case of a gene for herbicide resistance. The selectable marker gene, if carried on a transposon, should be in the loop region so it will be integrated into the chloroplast chromosome.

Desired Heterologous Gene

The desired heterologous gene can be any gene which is capable of being expressed in chloroplasts, and which encodes a protein which provides or enhances a beneficial feature of the plant. An example is a modified version of photogene 32. plants which are resistant to the effects of triazine herbicides encode a photogene 32 product which differs by one amino acid from that of susceptible plants. Transfer of such a resistance-encoding photogene 32 to the chloroplast genome of susceptible plants will confer resistance to plant cells containing the transformed chloroplasts.

Additional representative commercially available herbicides to which resistance can be induced include 2, 2 dichloropropionic acid ("Dalapon"), glyphosate (N-phosphono-methyl glycine; "Roundup"), cyanazine, cholosulfuron, L-phosphinothricin, atrazine, the imidazolines, and 6-chloro-N-ethyl-N-(l-methyl-ethyl)-l, 3, 5-triazine-2, 4, diamine ("Gesaprim 500L").

Mechanisms by which vector encoded protein-mediated herbicide resistance can be induced in plants according to the invention include the following:

Detoxification -This involves either enzymatically altering the herbicide molecule by removing a substituent to render the molecule non-toxic; cleaving the molecule; or adding a substituent to the molecule to render it non-toxic. The first mechanism, removal of a substituent, is the mechanism by which a bacterial dehalogenase inactivates, by removal of the chlorine atoms, the herbicide Dalapon. The bacterial plasmid carrying the gene for the dehalogenase has been isolated and is described in Beeching et al. (l983) J. Gen. Microbiol. l29, 207l. An example of the addition of a substitutent to detoxify a substance toxic to plants is the enzymatic addition of a phosphate group to kanamycin by aminoglycoside phosphotransferase, described above.

Modification of Herbicide Target -This involves introducing into the plant a mutant gene which codes for a form of the herbicide-targeted enzyme which is not susceptible to poisoning by the herbicide.

Increase in Target -This involves introducing into the plant a gene encoding the enzyme poisoned by the herbicide, to increase the amount of the enzyme produced by the plant.

Various bacteria are able to use these mechanisms to render themselves resistant to herbicides. These bacteria are a convenient source of herbicide resistance-conferring genes. Such genes are generally isolated from such bacteria (generally soil bacteria) by first selecting bacteria either able to grow on the herbicide as a nutrient source or able to detoxify the herbicide. The enzymes responsible for herbicide detoxification in these bacteria are charaterized and the genes encoding the enzymes are then isolated according to standard methods.

Examples of other desirable heterologous genes are enzymes participating in nitrogen fixation. Other desirable genes are those encoding enzymes which must function embedded in a lipopolysaccharide membrane, e.g., photosynthetic enzymes or nitrite reduction enzymes.

## Site for Desired Heterologous Gene

The site for the insertion of a desired heterologous gene is a site within the transposon loop at which an endonuclease can act to cut the vector for insertion of the heterologous gene. Preferably the site is unique in the vector, so that the endonuclease cuts the vector only in the desired location.

## Plasmid Construction and Transformation

Particular plasmids, in particular pFCC4I::Tn5#4 (Figs. Ia and b) were constructed as follows, beginning with the growing of maize.

## Growing of Maize

The first step was to soak 1000 ml seeds of Zea mays variety FRB73 in tap water for 6-16 hrs. The seeds were placed in vermiculite 6 cm deep in 4 planters (each 6" $\times$ 20") and saturated with tap water for 6-16 hrs, then drained. The seeds were treated with the fungicide, Captan, at 2 tsp. per 1000 ml of seeds, and planted densely in planters I cm deep. Growth conditions were maintained at 29°C, I6 hour day, for 7-I4 days until plants reached the 2-3 leaf stage. The plants were moved to the dark 48-72 hrs before harvesting to reduce starch. The plants were watered as needed, especially I-2 days before harvesting. Approximate yield was 300-I000 g leaves per 1000 ml seeds.

## Preparation of Chloroplasts

Working quickly in a cold room, all solutions and glassware were cooled to 4°C. Leaves were cut, weighed and minced with scissors, then rinsed in 4% bleach and extensively in distilled water (dH$_2$O). Approximately 200 g minced leaves were mixed with 800 ml MET+ (330 mM Mannitol; 50 mM Tris HCl, pH8; 5mM Na$_2$EDTA, pH8; 5mM beta-mercaptoethanol; 0.1% bovine serum albumin). The leaf mixture was homogenized in a blender at low speed, then at high speed with 2-3 second bursts. The homogenate was strained through I layer, then 4 layers of Miracloth. Samples were removed at this, and other appropriate points for microscopic observation. The chloroplasts were collected and centrifuged at 3000 rpm for I0 min in the 6 $\times$ 250 ml angle rotor at 4°C. The resulting pellet was retained and the procedure repeated with the supernatant. A second pellet was collected, combined with the first, and resuspended in MET+. Approximately I000 ml MET+ was used for every 200 g of leaves. The suspension was centrifuged in a swing-out rotor at speed #7 in an IEC clinical centrifuge for I0 min. The pellet was retained, resuspended in I00 ml MET+, and the procedure repeated.

## DNase Treatment

The pellet from the preceeding procedure was resuspended in 25 ml MMT+ (330 mM Mannitol; 50 mM Tris HCl, pH8; I0mM MgCl$_2$; 5mM beta-mercaptoethanol; 0.1% bovine serum albumin); and DNase was added to a final concentration of 25 ug/ml. The preparation was mixed gently, and incubated on ice for 30 min with intermittent gentle mixing. EDTA was added to a final concentration of 20 mM and the mixture centrifuged at speed #7 for I0 min. The pellet was washed twice with a total of 25 ml SEM (330mM Mannitol; I50mM NaCl; 50mM Na$_2$EDTA, pH8). A DAPI test for chloroplast DNA was then performed (Nature (I975) 253, 46I).

## Sucrose Gradient Centrifugation

A continuous sucrose gradient in TE (50mM Tris HCl, pH8; 0.25mM Na$_2$EDTA, pH8), pH8, was prepared by first making a 45% sucrose solution in TE and dispensing it in 33 ml aliquots in polyallomer tubes. The tubes were frozen solid at -20°C and thawed at 4°C just prior to use to form the gradient. The chloroplasts were resuspended in I2-36 ml TE. A 3 ml layer of chloroplast suspension was placed on top of each gradient, and the tubes topped up with additional TE. The tubes were centrifuged at 27,000 rpm. for 60 min. at 4°C using a SW28 rotor. All of the large green upper phase (I0-20 ml.) was removed (avoiding the lower green particulate band and white pellet) and diluted about I:I0 with TE. The mixture was centrifuged at speed #7 for I0 min. to pellet the chloroplasts and wash away the sucrose. The pellet was resuspended in a smaller volume of TE and centrifugation repeated.

## Chloroplast Lysis for DNA purification

The pellet from the preceding procedure was resuspended in 8-32 ml TE, and 10% Sarkosyl added to a final concentration of 2%. The suspension was gently shaken to mix, and incubated on ice for 5-10 min until lysis.

## cpDNA Purification

To the lysate from the preceding procedure, 1 g Per ml of CsCl was added and allowed to dissolve 1 hr at room temperature, or overnight at 4°C. The chlorophyl was extracted 2-3 times with isopentyl alcohol until clear, and centrifuged at speed #5 each time to separate the phases and form the protein pellicle at the interphase. 0.8 ml of EtBr (10 mg/ml) was added and the refractive index adjusted to 1.393. The preparation was placed in medium-sized quickseal tubes, and filled with CsCl solution having the same index of refraction. The tubes were centrifuged in a Beckman centrifuge at 44,000 $\times$ g for 40-65 hrs in a Ti70.1 rotor at 15°C. The DNA band was collected, the EtBr extracted with isopentyl alcohol, and the DNA dialyzed with 8 liters of $T_5E_{0.25}$ (5mM Tris HCl, pH8; 0.25mM $Na_2EDTA$, pH8) at 4°C. The DNA solution was collected and the O.D. read to determine concentration. The DNA was run out on agarose gel; the size of the cpDNA was approximately 130 kb. The average yield of DNA was 50 ug cpDNA per kg leaves.

## Cloning cpDNA

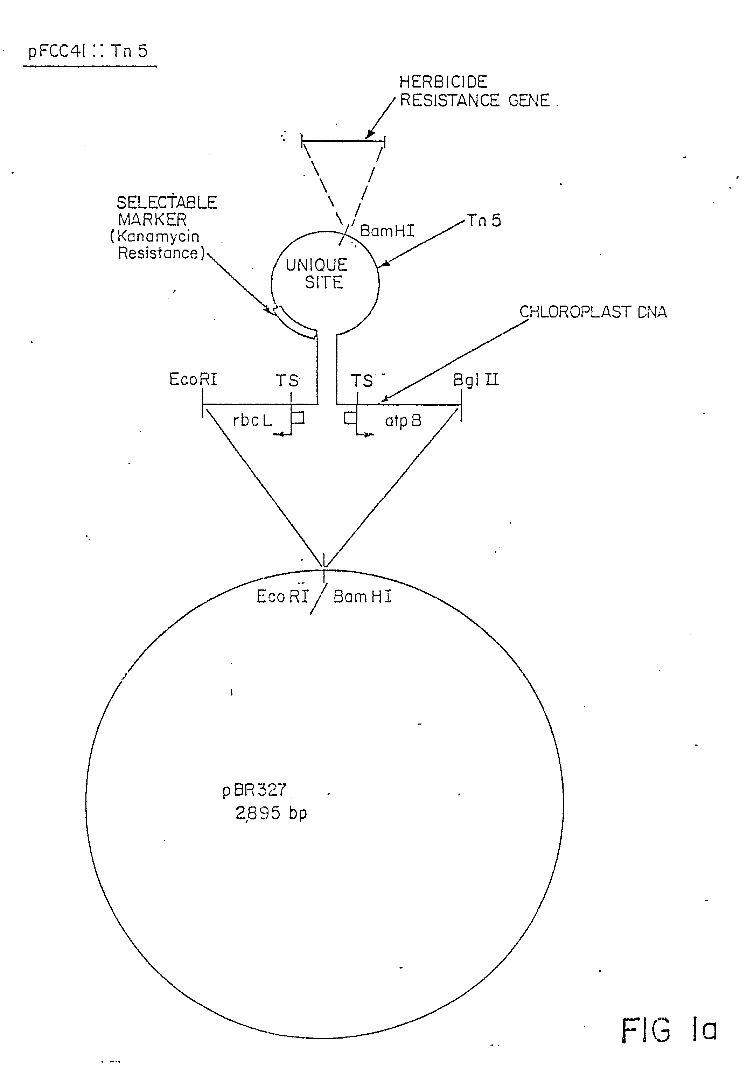

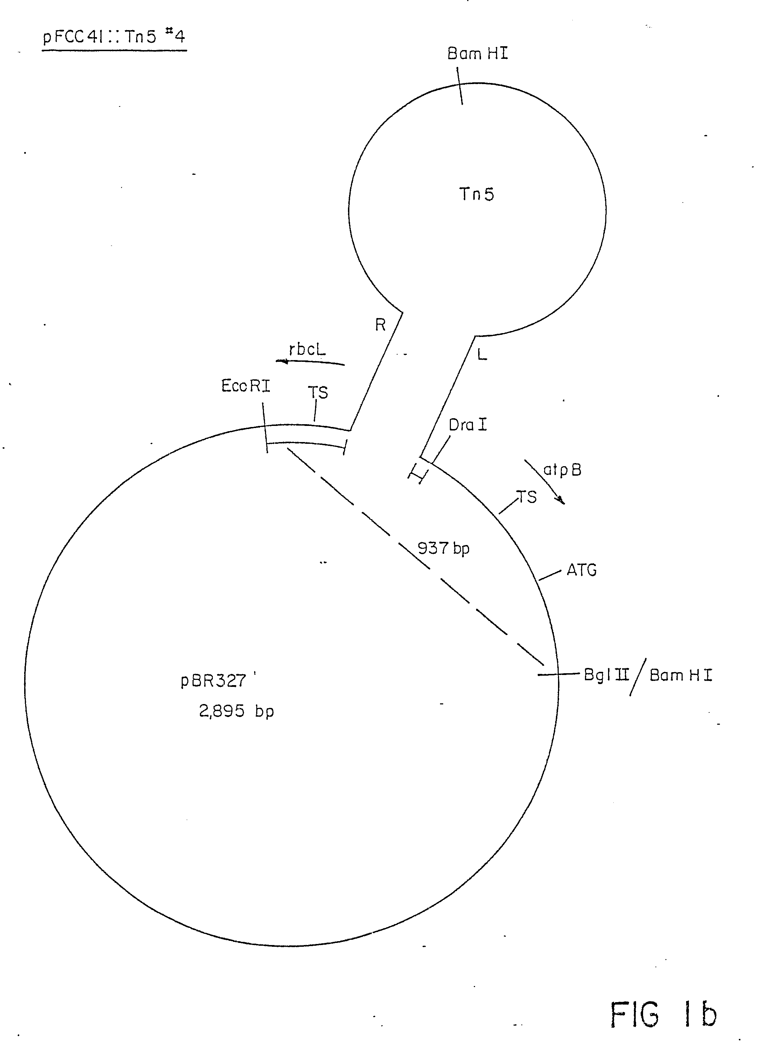

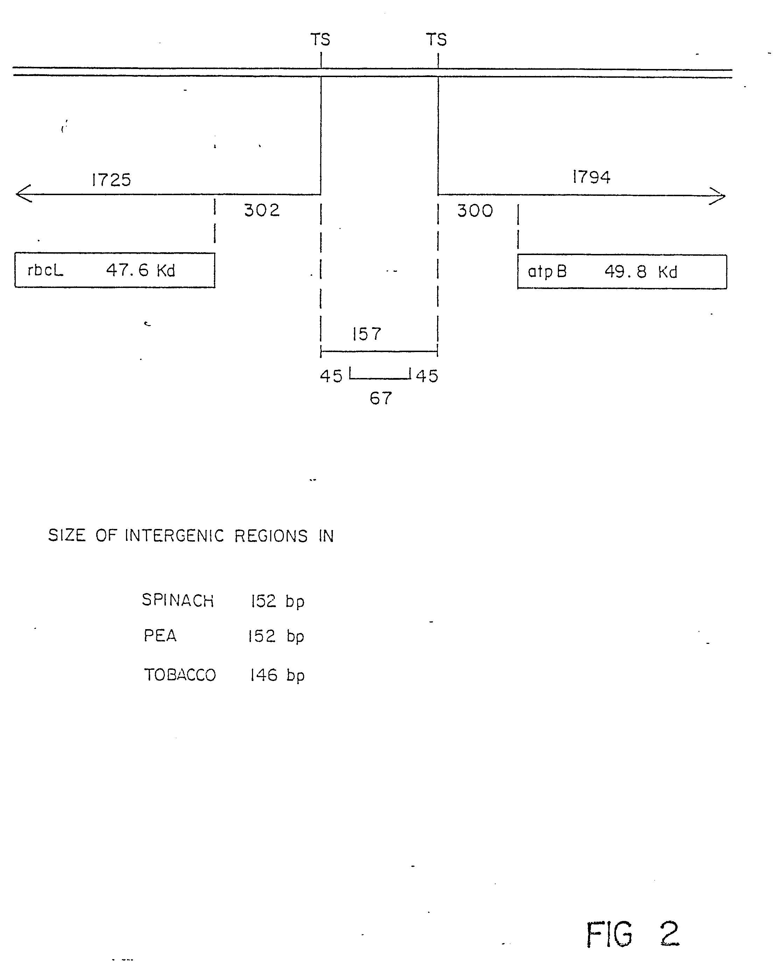

We chose a sequence on the maize chloroplast genetic map suspected of containing a transcriptionally silent region: the region between the transcriptional start sites of the gene for the large subunit of ribulose biphosphate carboxylase ("LS RUBISCO", rbcL), and the beta-ATPase gene (atpB), as shown in Fig. 2. This sequence is located on the BamHI 9 fragment of the chloroplast genome, which is, in turn, entirely contained on the SalI A fragment. We therefore chose to clone the BamHI 9 fragment by first cloning the SalI A fragment.

## Cloning of the SalI A Fragment of cpDNA

Purified cpDNA was digested with SalI and ligated to pBR327 which had been dephosphorylated, in a vector-to-fragment ratio of 2:1, followed by transformation of E. coli GM4. One of the inserts exhibited the expected size of the A fragment (28 kb); the plasmid carrying this insert (and another, 8.9 kb insert) was designated pAE.

Plasmid pAE was cut with SalI and the fragments religated. This yielded plasmids with fragment sizes corresponding to Sal A (pA) and Sal E (pE). The latter was confirmed as carrying the Sal E fragment and pA confirmed as carrying the Sal A fragment, by the Bam HI restriction mapping procedure described below.

## Subcloning of the BamHI 9 Fragment of cpDNA

Plasmid pA was cut with SalI and the presumptive Sal A fragment was purified by agarose gel electrophoresis. This fragment was cut with BamHI and the fragments generated were subcloned and confirmed to be correct. In addition to the BamHI fragments expected--namely, Bam 9, 9′, 10, 15′, 18, 19, 22′--we also consistently obtained BamHI fragments at 870 bp and at 500 bp. These were also cloned on pACYCl84.

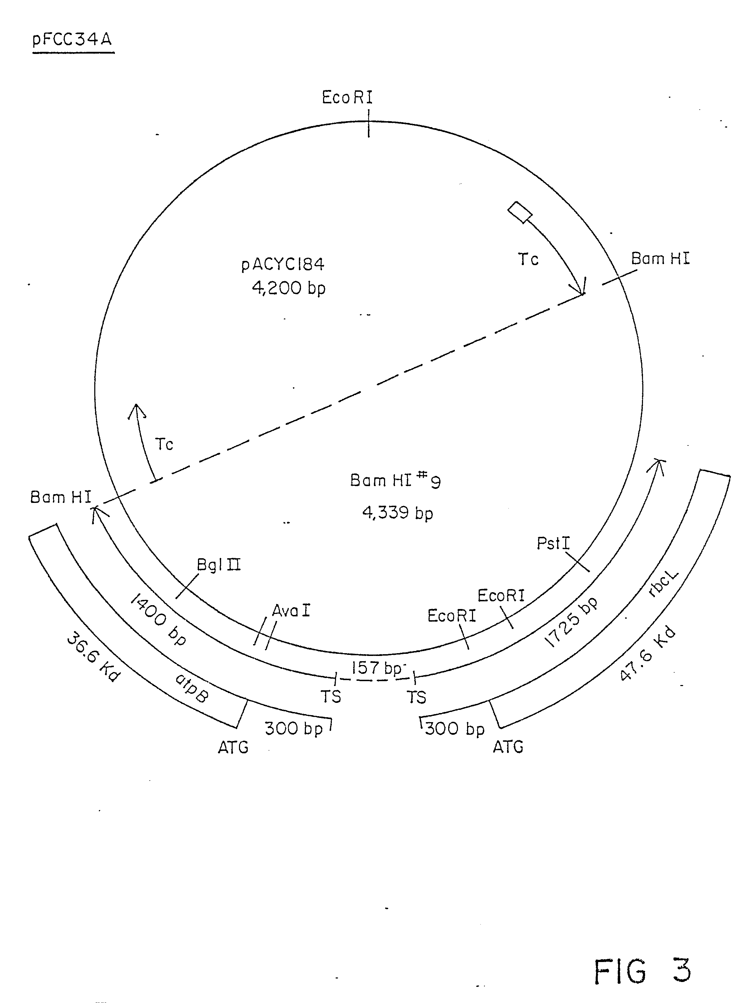

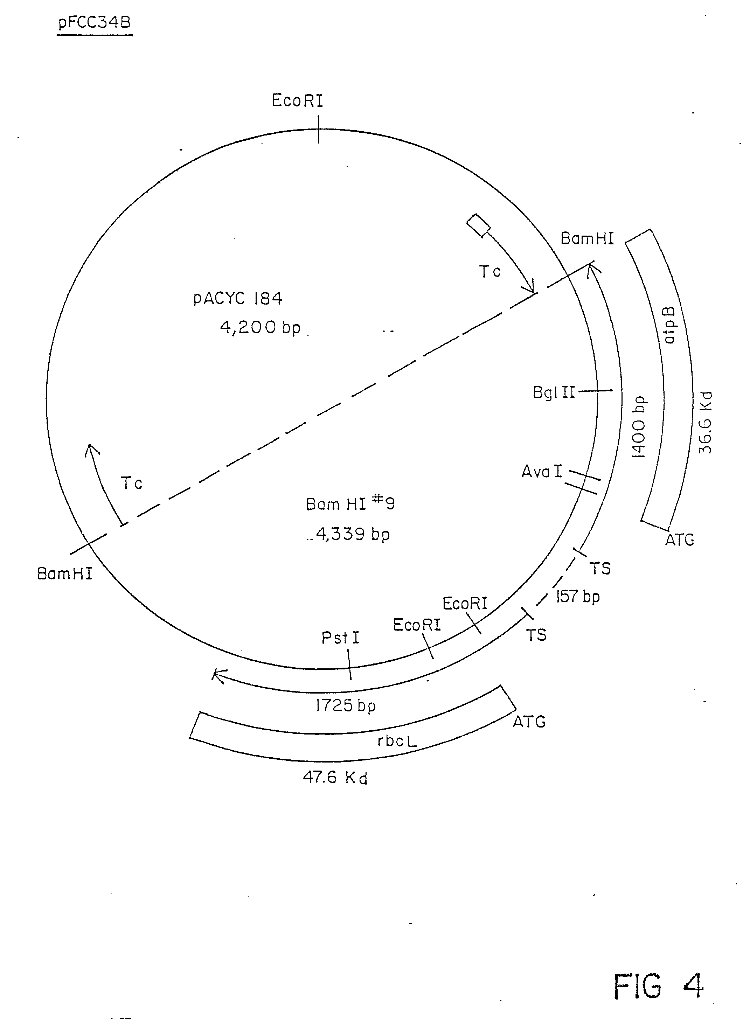

The BamHI 9 fragment was cloned into the BamHI site of pACYCl84 (Chang et al. (1978) J. Bacteriol. 134, 1141) in both orientations, yielding plasmids pFCC34A and pFCC34B (Figs. 3 and 4). This BamHI fragment carries the LS RUBISCO gene and its promoter. It also carries 1098 base pairs (encoding for a 36.6 kd polypeptide) of the 1494 base pair (encoding for a 54 kd polypeptide) beta subunit of ATPase and its promoter. The 157 base pair DNA sequence between these two structural genes carries the promoters for the two genes and a short sequence of apparently non-transcribed, non-essential DNA. This is the target sequence in the cp genome for integration of foreign genes.

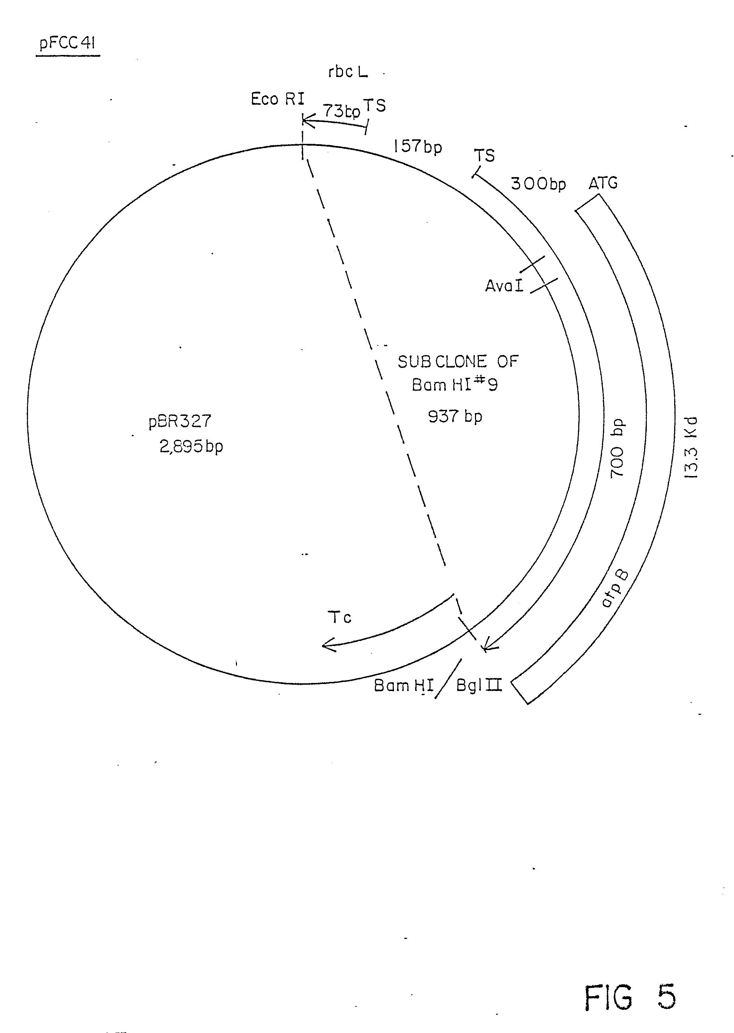

The following subclone was made from pFCC34B. The 937 base pair EcoRI to BglII fragment of the BamHI 9 fragment was cloned into the EcoRI to BamHI sites of pBR327, giving plasmid pFCC41 (Fig. 5). The subcloned fragment contains the transcriptionally silent intergenic region and the transcriptional start sites and other portions of the atpB and rbcL genes.

## Insertion of the Transposon into pFCC4I

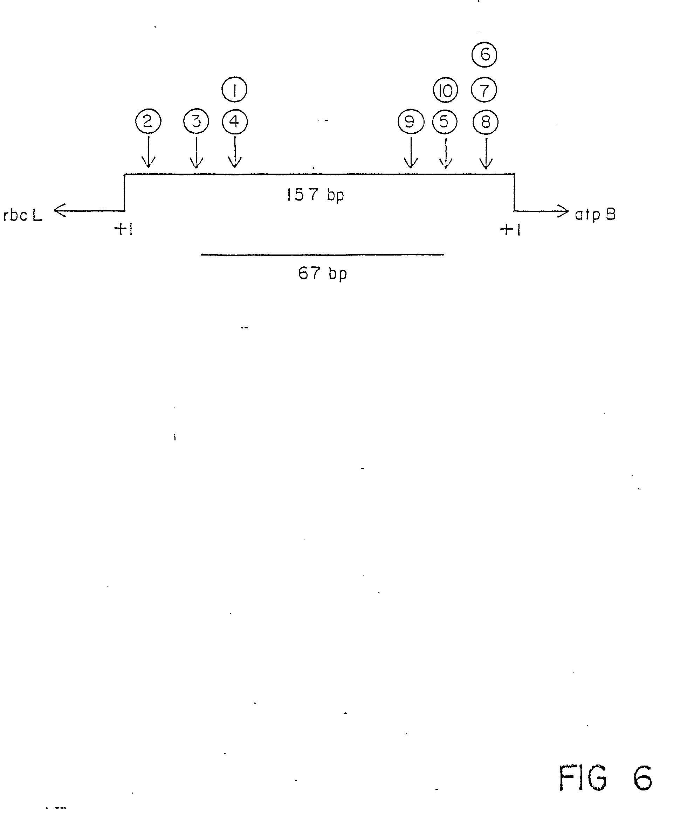

In experiments with E. coli, we discovered that the transposon Tn5 homogenitizes at a high frequency. We therefore chose to insert Tn5 into pFCC4I by homogenitization, by growing the plasmid in an E. coli strain bearing Tn5. In this way, we could select for molecules in which Tn5 had inserted into the silent region of the cpDNA. Plasmid pFCC4I was transformed into E. coliUNF5I0, a Tn5-bearing strain. UNF5I0 was plated on Luria agar containing I mg/ml kanamycin and I00ug/ml ampicillin. The colonies which grew on overnight incubation at 37°C were subcultured and their DNA was isolated by a mini-prep boiling method. Following restriction digestions by EcoRI and Xhol, the DNA was run on agarose gels in Tris acetate buffer. Of 700 clones tested, 426 of the plasmids carried Tn5, but only 300 of these had Tn5 located in the cpDNA insert. Restriction mapping of these clones identified I0 clones in which the transposon had inserted close to or in the transcriptionally-silent region of the cpDNA insert, and these were chosen for further study. The clones into which Tn5 had inserted have been called pFCC4I::Tn5 (Fig. IA), and the I0 clones of interest were numbered I to I0. Fig. 6 shows the approximate location of the Tn5 insertion in each of these I0 clones, within the I57 bp region between rbcL and atp B. Herbicide resistance encoding DNA may also be inserted into Tn5, as shown in dotted line, in Fig. IA.

## Analysis of Tn5 Inserts

Uncut pFCC4I::Tn5 plasmids when electrophoresed on agarose gels showed various multimeric forms but no monomeric forms. When cut with restriction enzymes, these plasmids have bands at 9.5 kb and 3.8 kb, the respective sizes of pFCC4I::Tn5 and pFCC4I.

Tn5 did not spread throughout the entire plasmid population by homogenitization as one might expect. After ten subculturings, going each time from isolated colonies while selecting for APH II, and also ten subculturings via I $\times$ I0$^{-4}$ dilutions, both populations of plasmid, i.e., pFCC4I and pFCC4I::Tn5, still existed in approximately equal portions. These results were obtained both in E. coli strains MM294 and GM4 (recA).

The mixed populations of pFCC4I and pFCC4I::Tn5 were separated by cutting the DNA with EcoRI (which cuts each DNA molecule once); separating the plasmids by size on agarose gels; purifying pFCC4I::Tn5; diluting the DNA; ligating to self; transforming this DNA into E. coli; and selecting for kanamycin resistance and ampicillin resistance. All of the clones resulting from this method were pFCC4I::Tn5 (one plasmid population only). They are very stable and exist in GM4 and in MM294.

The I0 candidate clones were further studied to determine which insertions did not hinder transcription of the rbc L and atpB genes. All ten clones have been sequenced to identify the site of insertion. Furthermore, a number of methods were utilized to test the effect of the insertions on transcription of the genes, including SI mapping, an in vitro transcription/translation assay, and assays for a chloramphenicol resistance gene that we inserted downstream from the rbcL gene on pFCC4I::Tn5. From these studies, two plasmids, #I and #4, with Tn5 insertions at positions -2I/-22 and -40/-4I (measured from the rbcL transcriptional start site), respectively, have been shown not to interfere with transcription of either the rbcL gene or the atpB gene.

## Tobacco Vectors

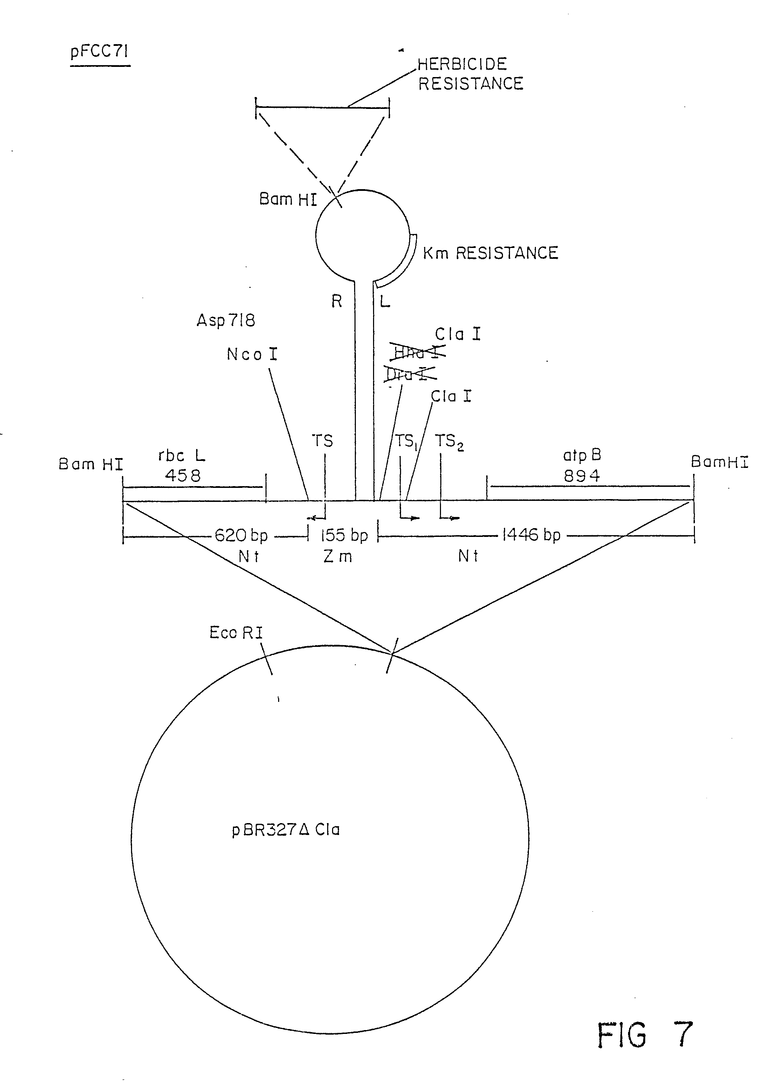

Referring to Fig. 7, plasmid pFCC7I is a vector specifically designed to transform choroplasts of tobacco plants. pFCC7I is similar in structure to pFCC4I::Tn5 #4, except that it contains DNA from the chloroplast genome of Nicotiana tabacum - (tobacco), which enables integration of the vector into the N. tabacum chloroplast genome. As in pFCC4I::Tn5 #4, transposon Tn5 is inserted into the transcriptionally silent region of the Z. mays chloroplast genome, but in pFCC7I, this silent region is flanked by N . tabucum sequences from the rbcL and atpB genes. The Z. mays silent region is closely homologous to the N. tabacum silent region: hence the entire hybrid DNA sequence on pFCC7I is suitable for directed integration into the tobacco chloroplast genome.

pFCC7I was derived from pFCC4I::Tn5 #4, which provided the Z. mays transcriptionally silent region, the Tn5 transposon containing the kanamycin resistance gene and a unique BamHI site for insertion of a desired heterologous gene, and from PEHA, which is a storage vector for the N. tabacum sequences. The construction of pFCC7I is described below, beginning with the growing of tobacco.

## Growing of Tobacco

The first step in constructing a vector for transforming tobacco chloroplasts was the growing of tobacco. Nicotiana tabacum (Ms Burley X Kyl0) was grown in a growth cabinet in Cornell peat-lite Mix for a total time of I2-I6 weeks at 28°C/I6 hours

light, 16°C/8 hours dark. The seeds were planted in shallow trays and at one month the most healthy young plants were transferred to one plant per pot. The leaves were harvested when the largest measured about 6"×4" (15.24cm × 10.16cm) and the plants were about 18" (45.72cm) high. The plants were placed in the dark for 2 days prior to harvesting to reduce starch content.

### Isolation of TobaccoChoroplasts and Tobacco cp DNA

Tobacco chloroplasts were isolated, and DNA from the chloroplasts isolated, according to the procedures described above for maize.

### Cloning Tobacco cp DNA

We chose to isolate a sequence from the tobacco chloroplast genome corresponding to the maize sequence described above. This sequence contains the presumptive transcriptionally silent region between the rbcL and the atpB genes, as well as flanking sequences containing the transcriptional start sites and 5' ends of the genes. In tobacco, this sequence is contained on a 2.1 kb BamHI fragment which, in turn, is entirely contained on the 15.2 kb SalI 6 fragment. We therefore chose to clone the BamHI fragment by first cloning the SalI 6 fragment.

### Cloning of the SalI 6 Fragment of Tobacco cp DNA

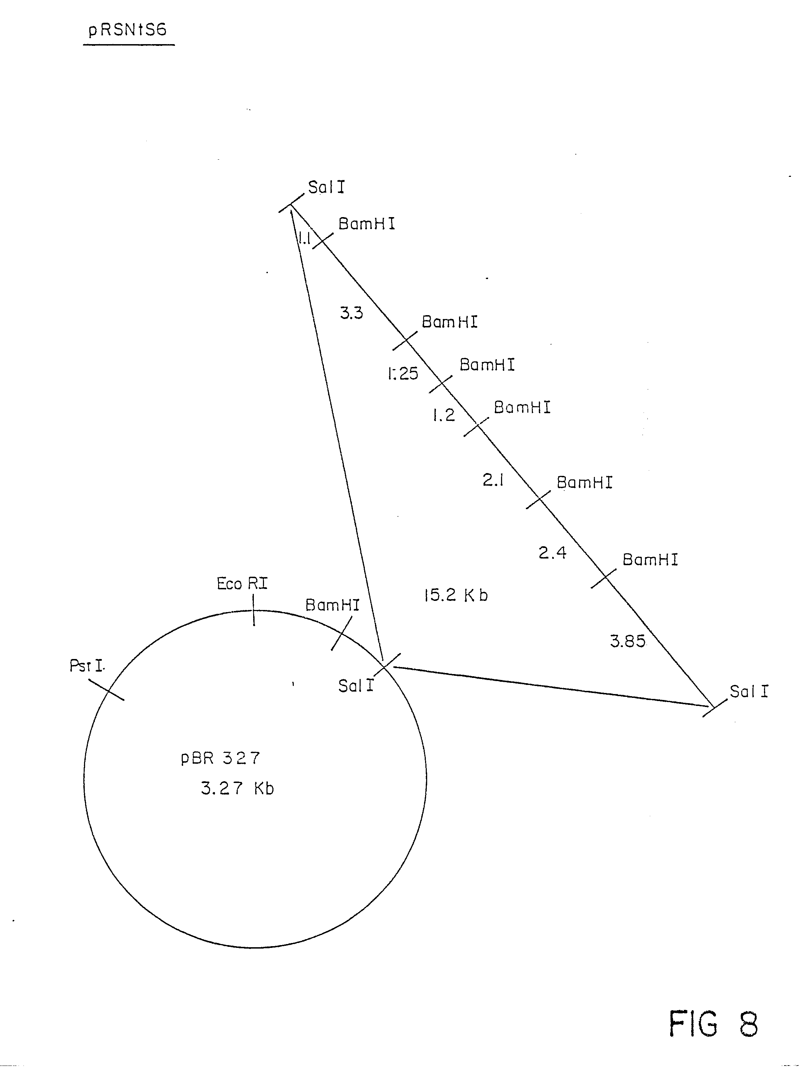

Purified tobacco cp DNA was digested with SalI and cloned into the SalI site of pBR327. When mini-preps were analysed on gels, five of the expected eleven SalI fragments were identified. These were fragments 4, 6, 9, 10 and 11. The plasmid carrying the 15.2 kb SalI 6 fragment, pRSNtS6 (Figure 8), was subcloned as described below.

### Subcloning of the 2.1 kb BamHI Fragment ofTobacco cp DNA

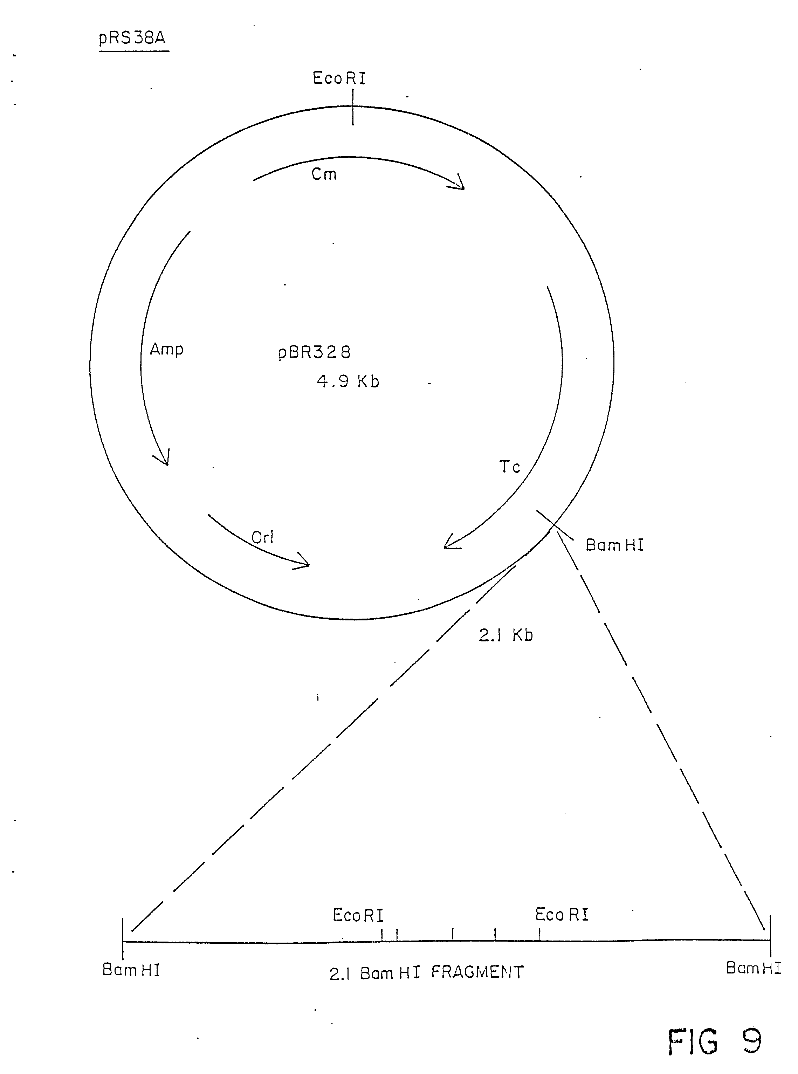

pRSNtS6 was digested with SalI, and the SalI 6 fragment purified by agarose gel electrophoresis. This fragment was digested with BamHI and the fragments generated subcloned into the BamHI site of pBR328. The seven expected BamHI fragments were all identified in the clones analysed, including the one of interest, the 2.1 kb fragment. The Plasmid containing this fragment is named pRS38A (see Figure 9).

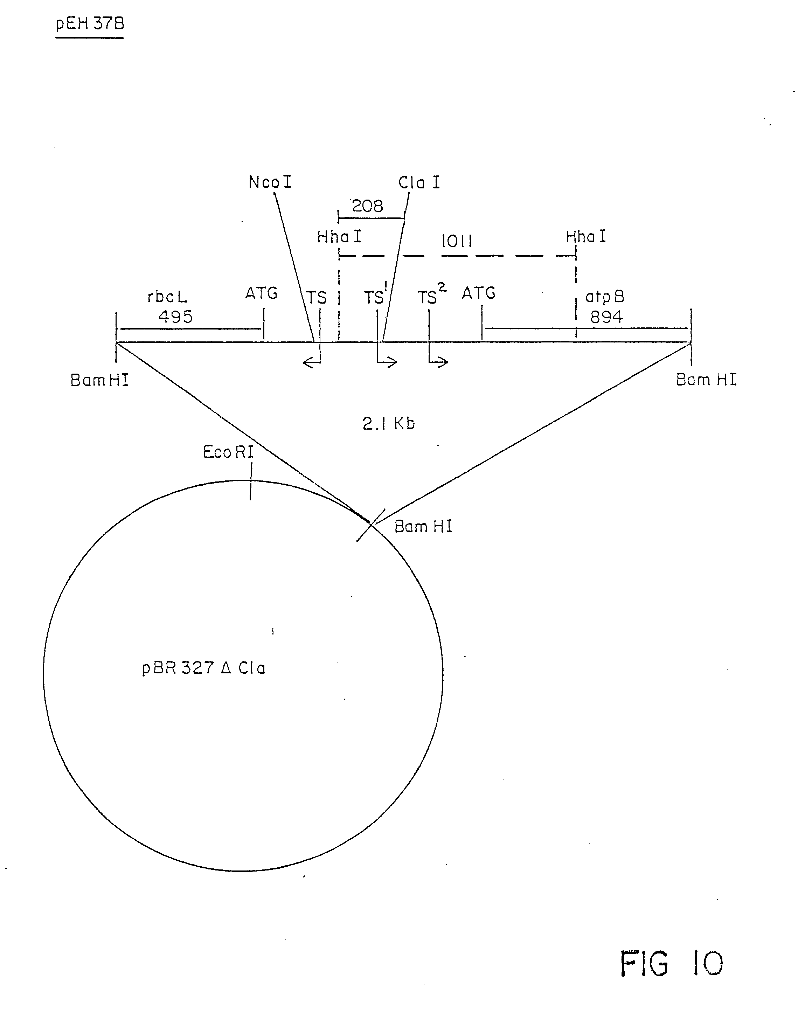

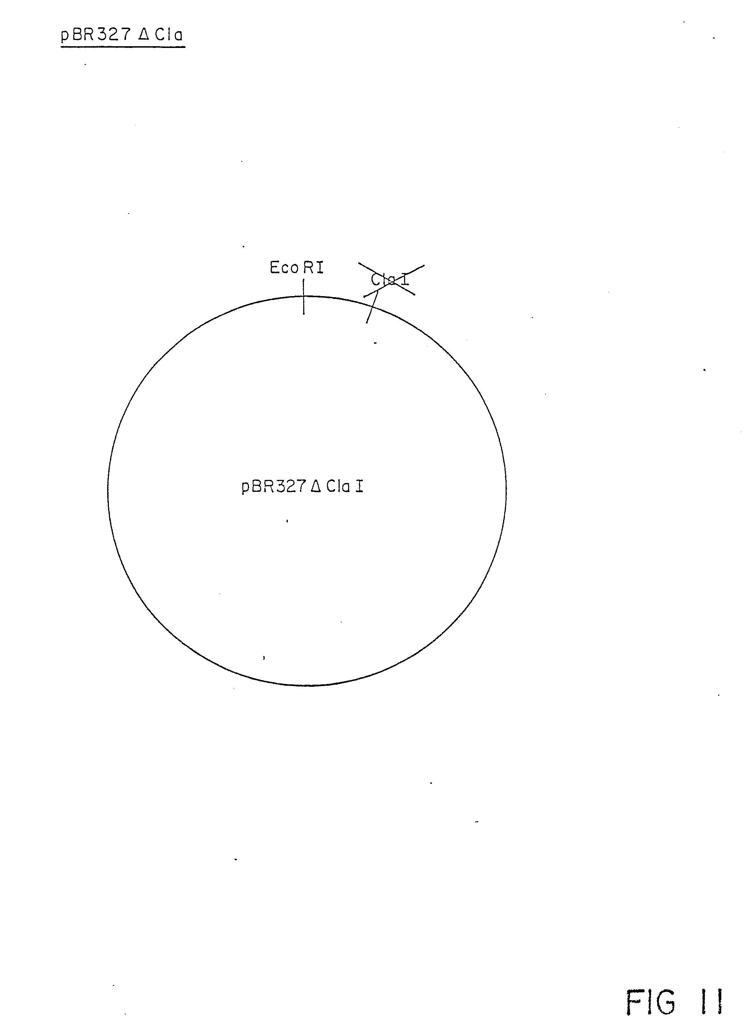

Next, PEHA (Fig. 10) was constructed, as follows. Plasmid pBR327Δ Cla (Fig. 11) was made by cutting pBR327 with Cla I, filling the ends in with Klenow polymerase, religating, and screening for a clone with a deleted ClaI site. Next, the 2.1 kb BamHI fragment from pRS38A (Fig. 9), was cloned into the BamHI site of pBR327 Δ Cla to yield PEHA, a storage vector for the desired fragment. TS[1] and TS[2] are two potential transcriptional start sites for atpB: TS[2] has been previously described and TS[1] has been identified by our own sequence data. The transcriptional start site for the rbcL gene is also included on this subclone.

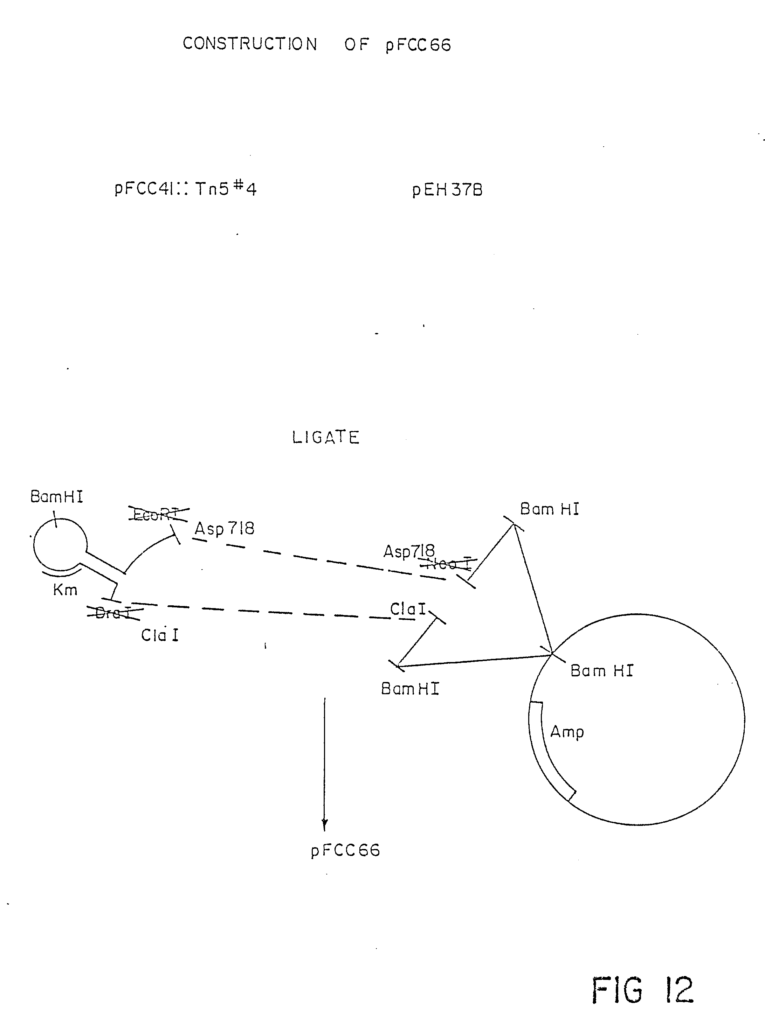

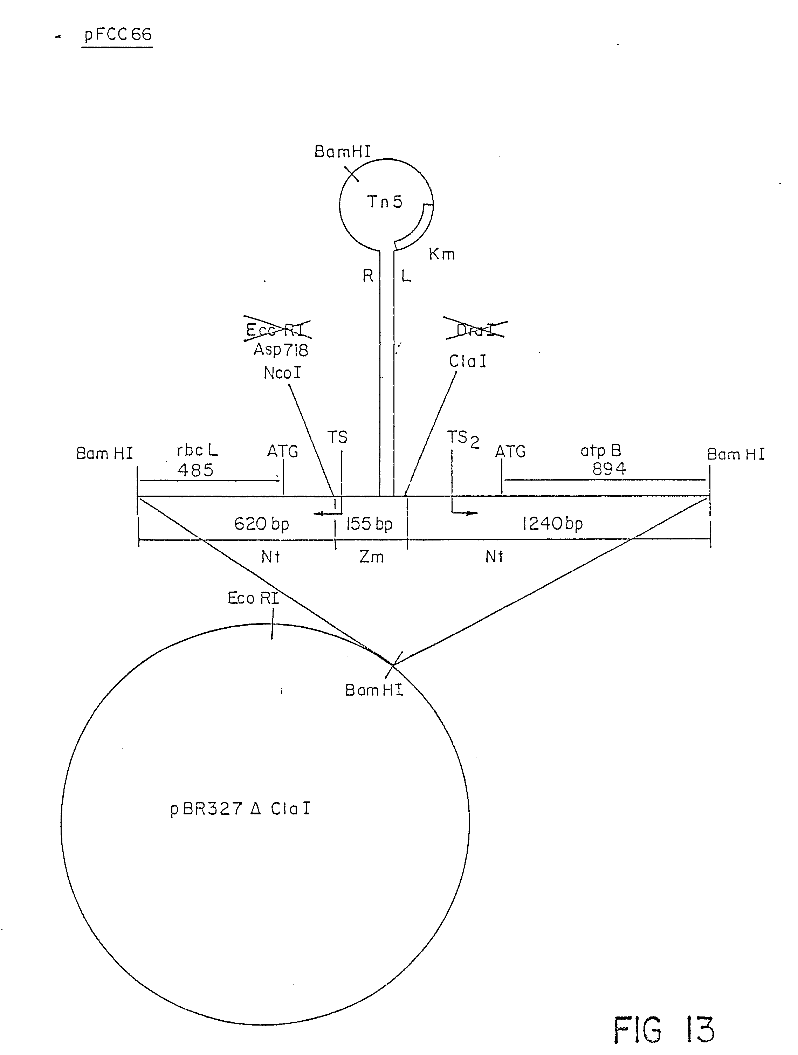

To construct pFCC66 (Fig. 12 and 13), pFCC41:Tn5 #4 was cut with EcoRI, the ends were filled in with Klenow polymerase, and the filled-in end ligated to an Asp718 linker (GGGTACCC). The vector was cut on the other side of Tn5 with DraI, ligated to a ClaI linker, and the Tn5-containing fragment was then cut out with Asp718 and ClaI and purified. PEHA (Fig. 10) was cut with NcoI, the ends filled in with Klenow polymerase, ligated to an Asp718 linker, cut with Asp718 and ClaI, and purified. The Tn5-containing fragment from pFCC41:Tn5 #4 was inserted into the backbone of PEHA as illustrated.

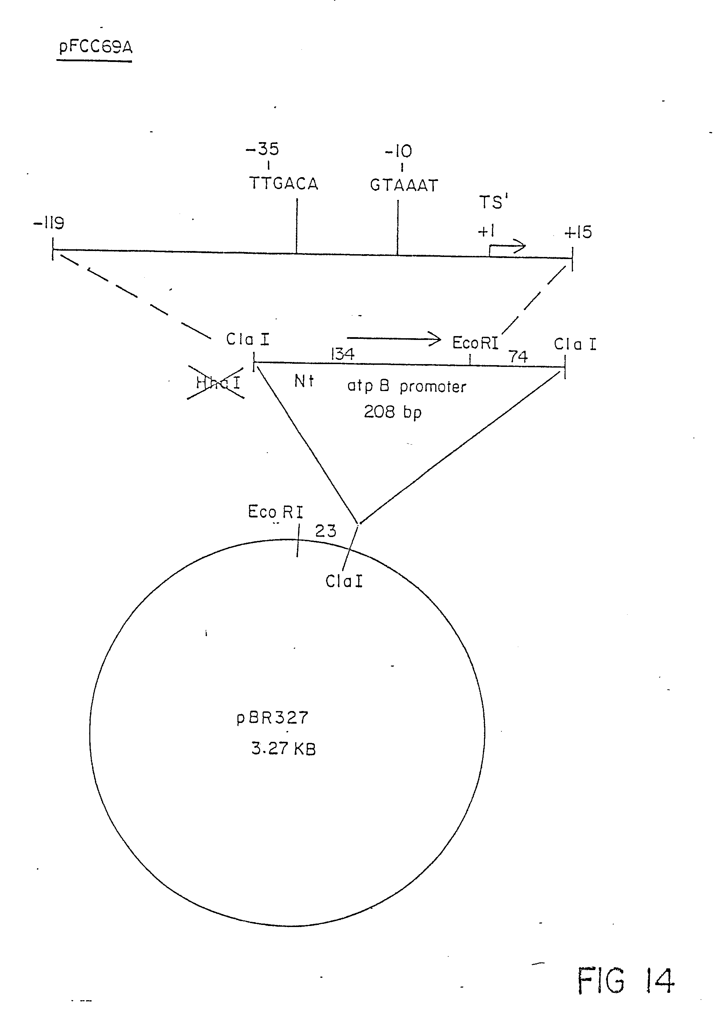

In constructing pFCC66 (Fig. 12), one of the two transcriptional start sites (the upstream: site, TS[1]) of the tobacco atpB gene was deleted. We have discovered this site to be potentially important in expression of atpB. In order to ensure proper expression of the genomic atp B gene in chloroplasts transformed by the vector, a 208 bp Cla I fragment of PEHA was inserted into the ClaI site of pFCC66 to yield pFCC71 (Figure 7). The 208 bp ClaI fragment was obtained by cutting PEHA (Fig. 10) with HhaI, purifying the resulting 1011 bp fragment, filling in with Klenow polymerase, ligating to ClaI linkers, and cutting with ClaI. The resulting fragments were ligated to ClaI-cut pBR327, yeilding the storage vector pFCC69A (Fig. 14), which contains the 208bp Cla I fragment. This ClaI fragment was excised from pFCC69A and ligated into the ClaI site of pFCC66 to yield pFCC71.

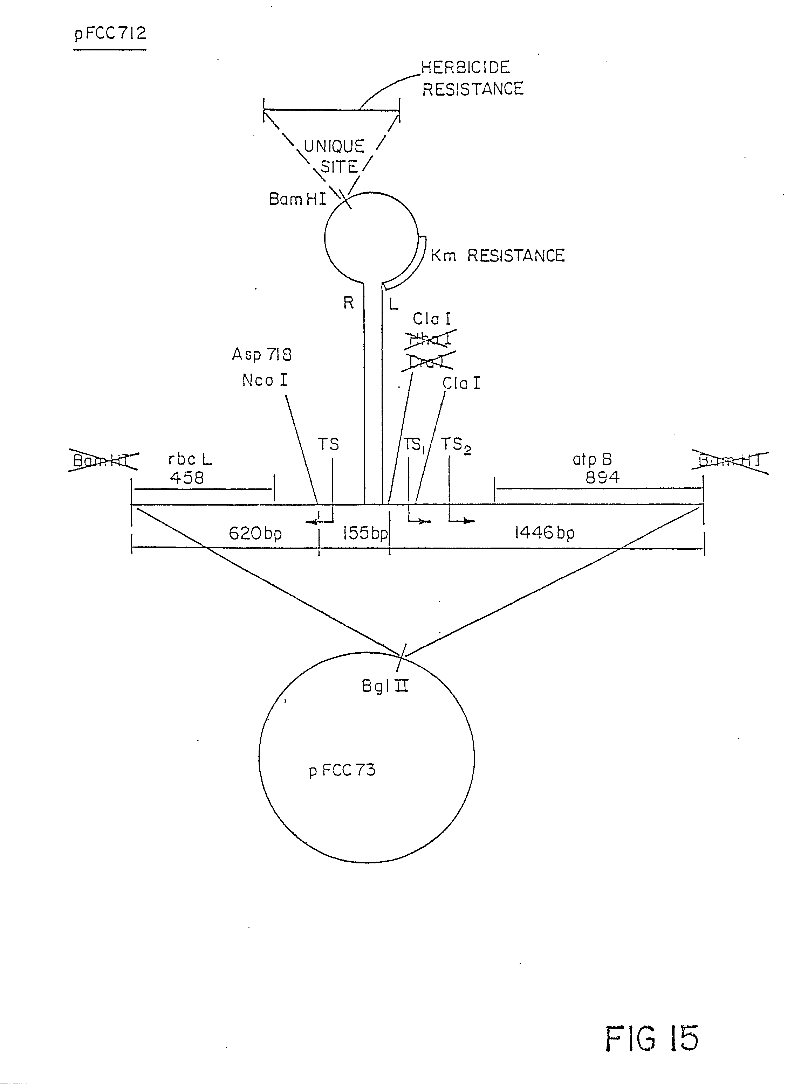

pFCC71 is a vector which can be used to transform chloroplasts in vivo by electroporation or isolated chloroplasts in vitro by electroporation or by permeabilizing the chloroplast's outer membrane with calcium. pFCC71 is not, however, a broad-host range vector, and thus cannot be used to introduce DNA into chloroplasts in intact plant cells via Agrobacterium (as is described in more detail below). To enable the use of this method, the relevant DNA was inserted into pFCC73, a derivative of the broad-host range vector pSUP104, to yield pFCC712 (Fig. 15), as follows.

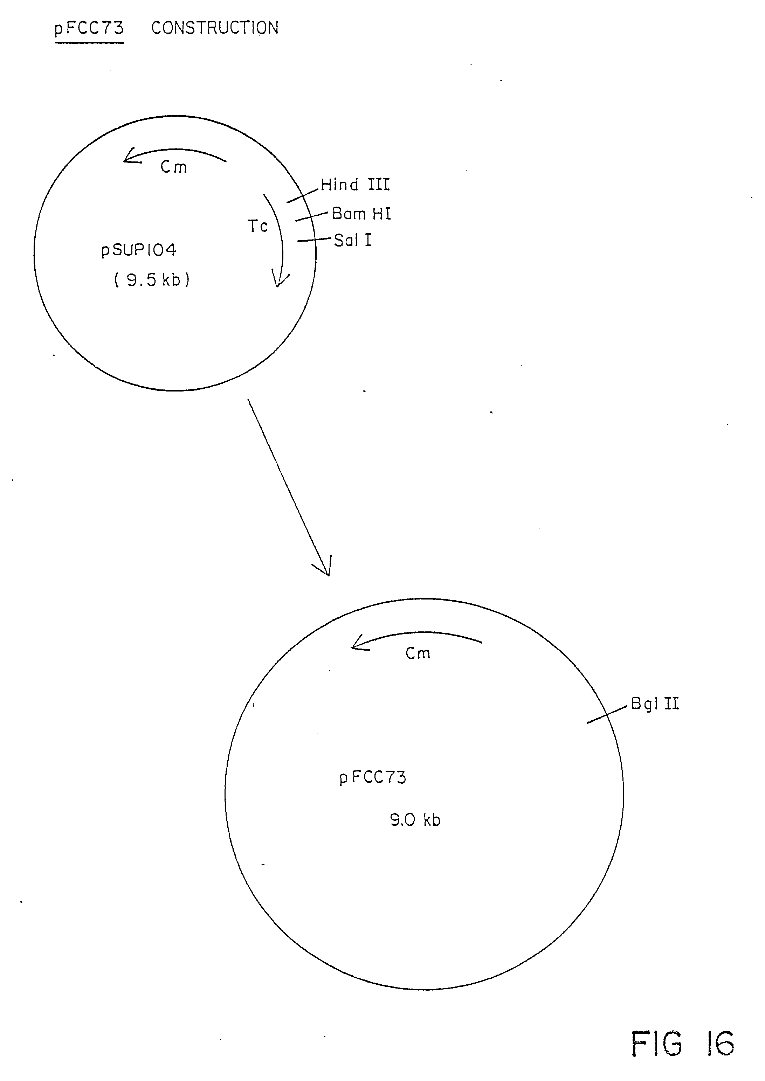

pFCC73 was constructed from pSUPI04 as shown in Figure 16. pSUPI04 was cut with HindIII and SalI; the ends filled in with Klenow polymerase and ligated to BglII linkers. The DNA was cut with BglII, diluted and then religated, resulting in pFCC73 as shown in Fig. 16.

pFCC71, which has three BamHI sites, was partially cut with BamHI, maximizing for 2 cuts, and cloned into pFCC73 (Figure 16) at its unique BglII site. The resulting plasmid, as shown in Figure 15, is pFCC712.

Random Integration and Autonomously Replicating Vectors

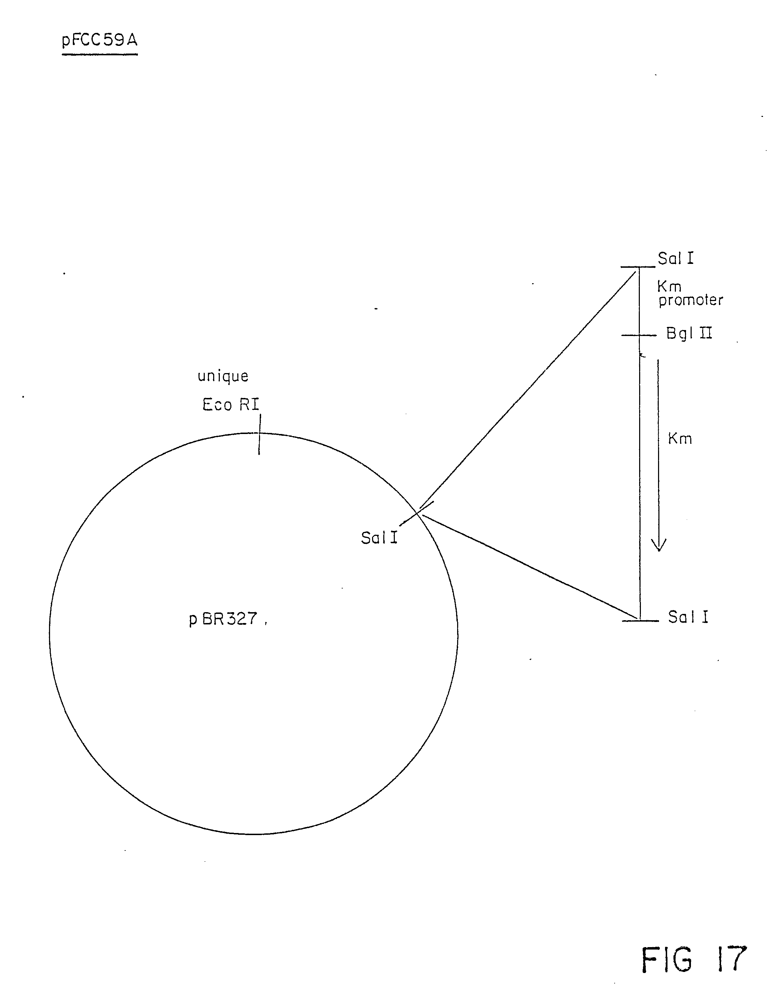

As is mentioned above, although direction of the heterologous DNA to a transcriptionally silent region of the chloroplast genome is in some instances desirable, it is not necessary. In Fig. 17 there is illustrated pFCC59A, constructed by inserting the kanamycin resistance gene-containing SalI fragment from Tn5 into the SalI site of pBR327. The unique EcoRI site on the vector can serve as the site for the insertion of a desired heterologous gene. pFCC59A can be linearized at a unique restriction site not located in the heterologous gene, and inserted into chloroplasts by electroporation, where it can integrate randomly into the chloroplast genome. Suitable restriction sites for linearization include the HindIII, BamHI, or EcoRI sites of pBR327.

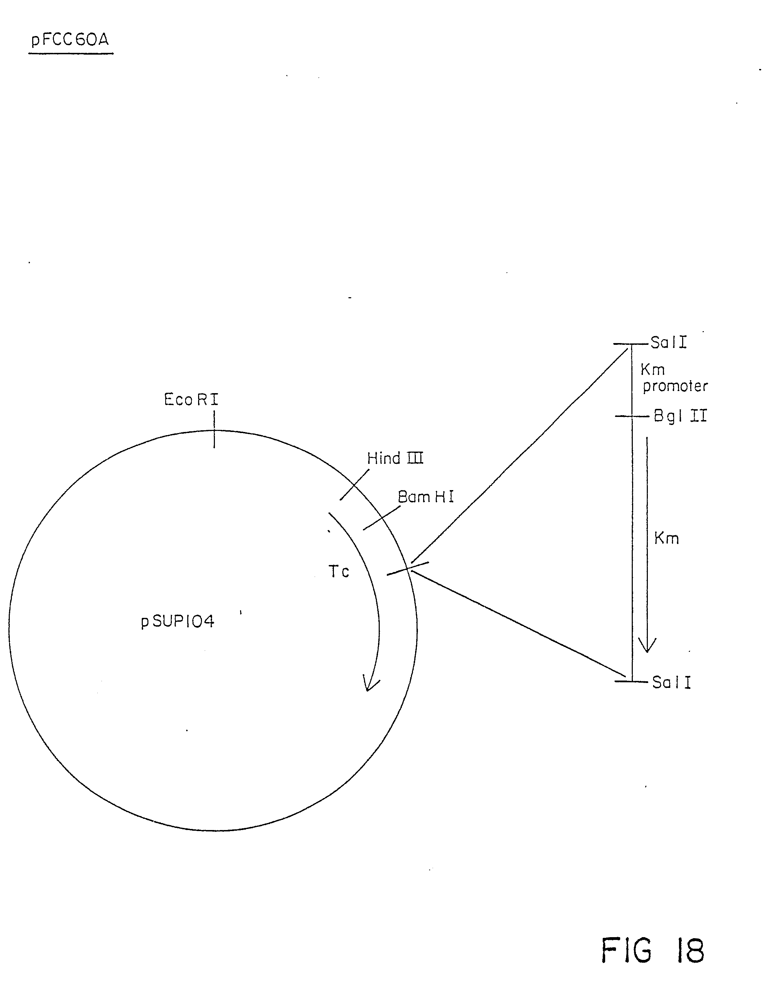

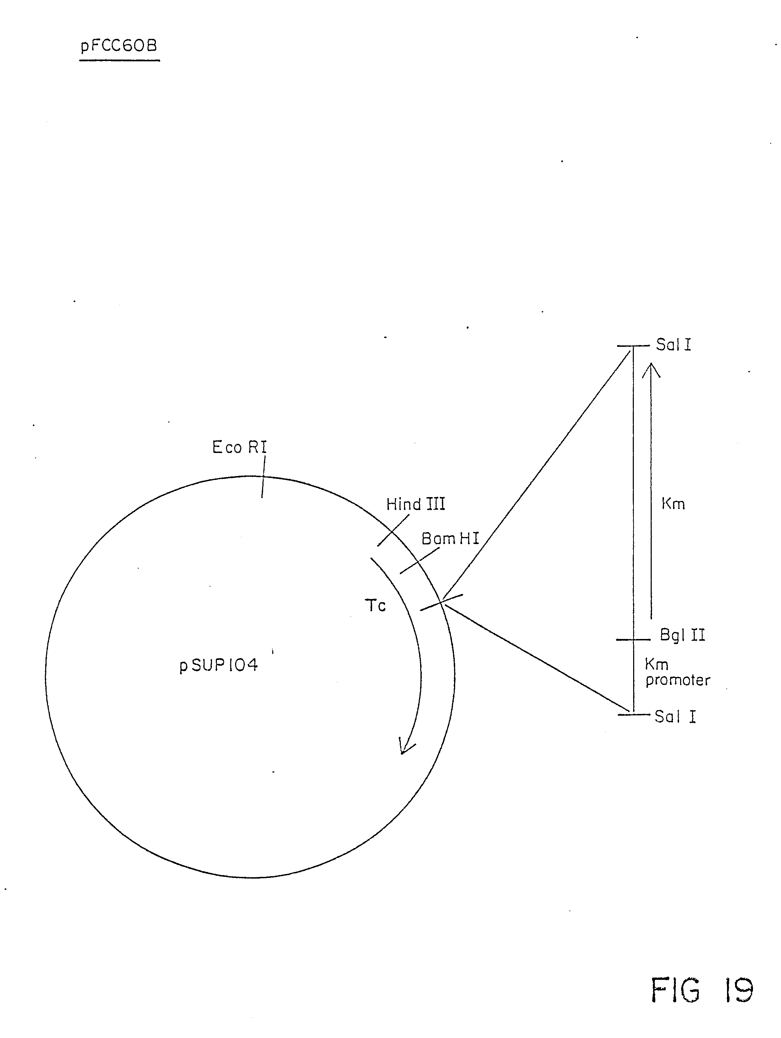

Referring to Fig. 18, pFCC60A contains the same fragment of Tn5 as pFCC59A, but inserted into pSUPI04. pFCC60A will autonomously replicate in chloroplasts, or can be integrated into the chloroplast genome randomly by linearization at one of the unique sites listed above. pFCC60B (Fig. 19) contains the Tn5 SalI fragment in the opposite orientation of pFCC60A.

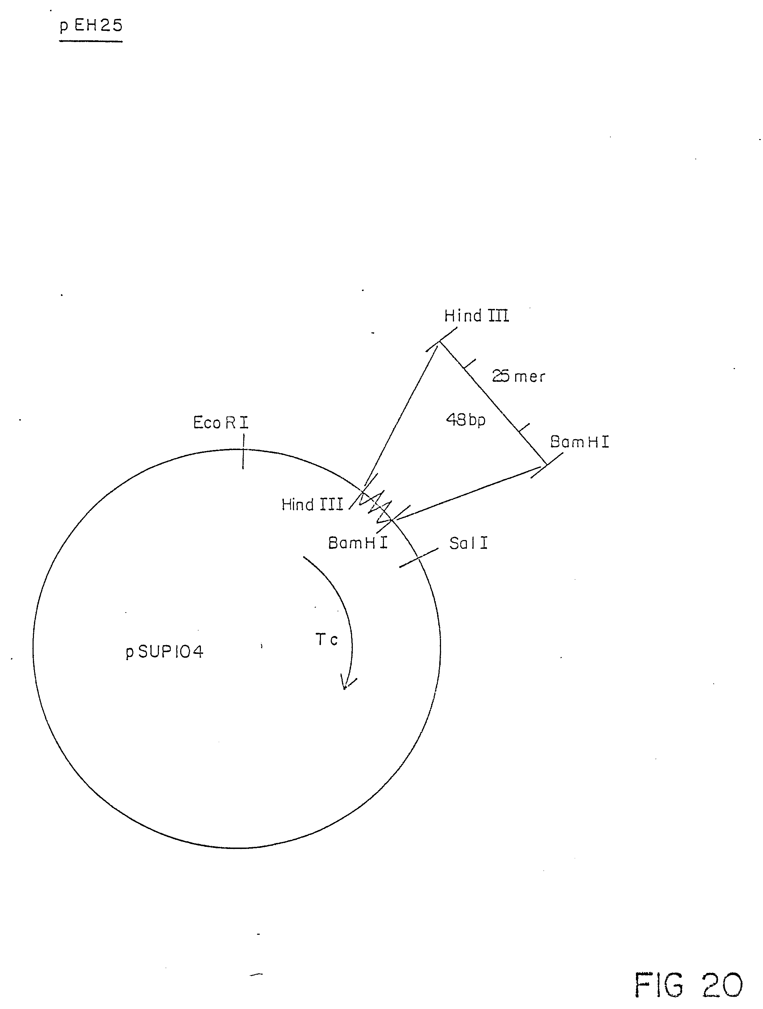

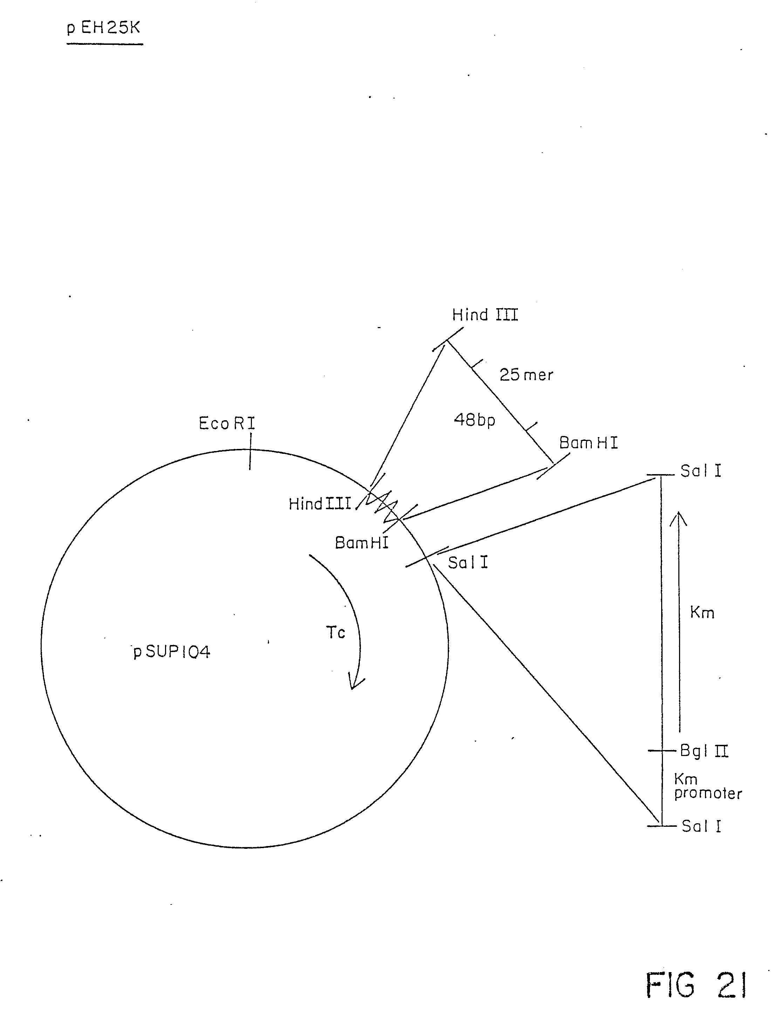

To facilitate integration of the desired DNA into the chloroplast genome, integrated Ti plasmid border DNA, or synthetic border DNA, can be employed, as described in our European Patent Application No. 86900506.6 (Publication No.    ), the disclosure of which is to be regarded as hereby incorporated by reference. Referring to Fig. 20, a 48 bp HindIII-BamHI fragment containing the 25-bp synthetic border fragment described in our above mentioned European Patent Application was inserted into pSUPI04 to yield pEH25. The above-described SalI fragment from Tn5 was then inserted into the SalI site of pEH25 to yield pEH25K (Fig. 21), which can replicate autonomously in chloroplasts, or integrate randomly into their genomes.

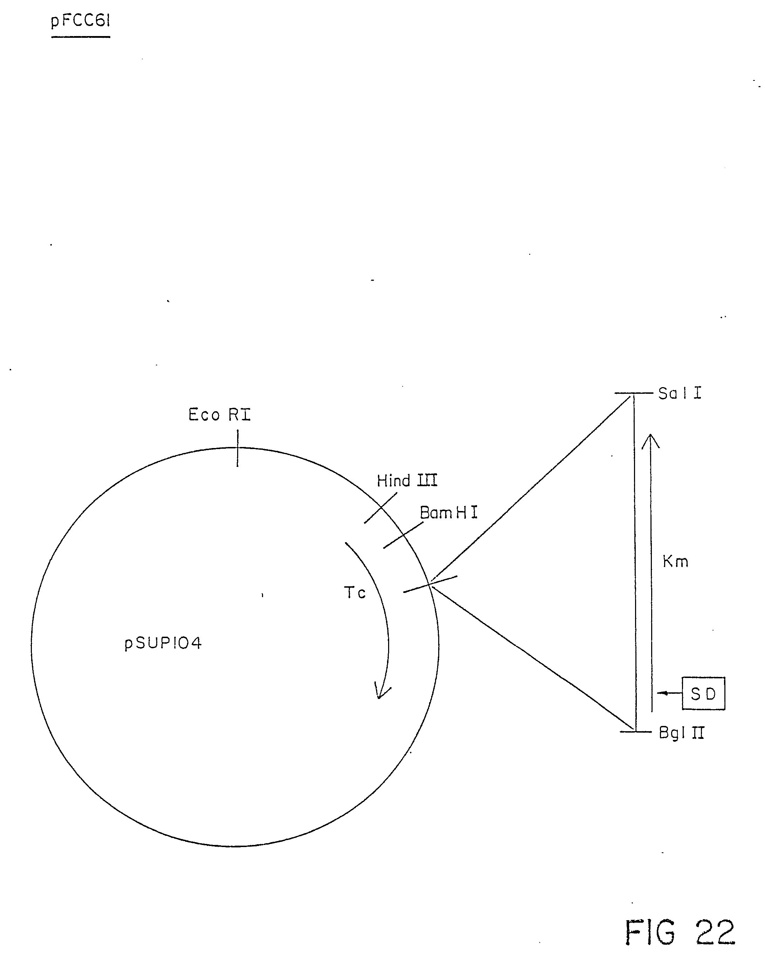

Referring to Fig. 22, pFCC61 contains a kanamycin resistance gene-containing fragment from Tn5, minus the kanamycin resistance gene's promoter, and including a BglII site for the insertion of a chloroplast promoter, e.g., the rbcL or atpB promoter, to more efficiently transcribe the kanamycin resistance gene. These promoters can replace the naturally-occuring promoter of the selectable marker gene of any of our vectors, and can also be used to control transcription of the gene for the desired heterologous protein.

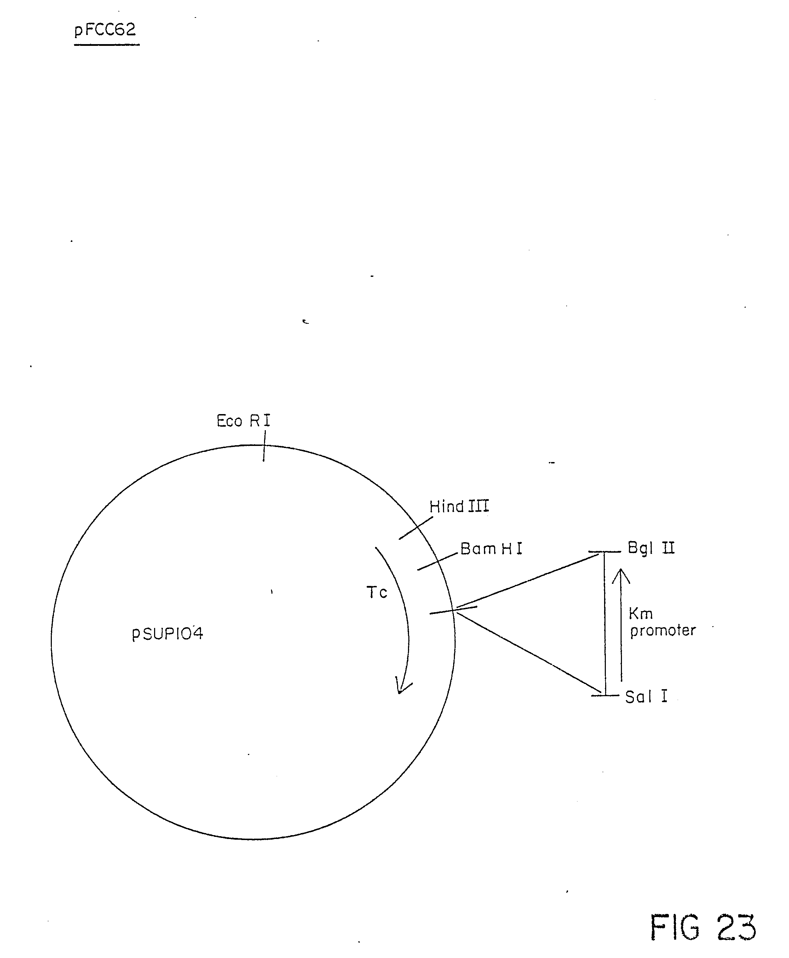

Referring to Fig. 23, pFCC62 contains the kanamycin resistance gene's promoter, but not the gene itself, which has been replaced by a BglII site for the insertion of a gene for another selectable marker, or the desired heterologous gene.

Other Vectors

For site-specific integration into chloroplasts of plant species not having sufficient homology to the silent regions of corn and tobacco chloroplasts, one skilled in the art will be able to construct vectors, utilizing transcriptionally silent regions of the desired chloroplast genomes, using methods analogous to those described above.

Insertion of Desired Heterologous Gene

The desired heterologous gene can be inserted into the above-described vectors at the BamHI site in the transposon-derived portion of these plasmids, or at some other convenient site.

Transformation of Chloroplasts

The above-described vectors for random integration and autonomous replication can be used to transform chloroplasts of any plant species. The above-described vectors for site-specific integration can be usd to transform chloroplasts of specific plant species. Plasmid pFCC4I::Tn5 #4 and its derivatives can be used to transform maize chloroplasts, and can also be used to transform sorghum and rice chloroplasts, which are known to have considerable homology to the atpB-rbc L silent region of maize. pFCC71 and pFCC712 and their derivatives can be used to transform tobacco chloroplasts. These vectors can also be used to transform chloroplasts of any plant species whose chloroplasts have sufficient homology to the silent regions of the maize or tobacco chloroplast genomes.

The vectors are used to transform isolated chloroplasts and/or proplastids in vitro either by electroporation or by permeabilizing the chloroplast's outer membrane with calcium. The chloroplasts can be inserted into a plant cell protoplast by, e.g., electrofusion. Transformants are selected by means of the selectable marker (i.e., kanamycin resistance).

As is mentioned above, it may not be necessary to isolate chloroplasts in order to transform them with these plasmids. A recent report (DeBlock et al. EMBO Journal 4: 1367, 1985) indicates that DNA introduced via an Agrobacterium tumefaciens delivery system can enter the chloroplasts of intact plant cells. To use this method, the relevant DNA must be transferred, as described above, to a broad-host range vector such as pRK290 or pSUP104, to enable replication in A. tumefaciens. The resulting plasmid is then transferred to an A. tumefaciens strain, e.g., LBA4404, which carries a Ti plasmid, pLBA4404, deleted of the T-DNA region, so that cocultivation does not result in plant tumor formation. (pLBA4404, and binary cocultivation, are described in Hoekema et al. (1983) Nature 303, 5913). pLBA4404 does, however, retain the native Ti "vir" (for virulence) functions which are essential for the transfer of the hybrid plasmid of the invention from A. tumefaciens LBA4404 to the host plant cell. The A. tumefaciens containing the hybrid plasmid is then cocultivated with plant cells to insert the hybrid plasmid into the plant cells, where the plasmid DNA becomes integrated into the chloroplast chromosome. Transformants are selected by means of the selectable marker.

Integration into the chloroplast genome occurs as described above. Chloroplast DNA, e.g., portions of the rbcL and atpB genes, on the vector direct the vector to the corresponding homologous regions on the chromosome. The Tn5 portion of the vector then inserts the desired DNA (the kanamycin resistance gene and the site for the insertion of a heterologous gene) into the transcriptionally silent region of the chromosome between the promoters of the rbcL and atpB genes. Alternatively, random integration can occur, or, in the case of autonomously replicating vectors, there can be no integration at all.

Plant Regeneration

Following selection of transformants, the plant cells (protoplasts or other cells) are cultured under conditions effecting the regeneration of mature plants. Such methods are known, e.g., for the regeneration of tobacco plants from callus culture. See, for example, "Plant Tissue Culture: Methods and Applications in Agriculture", Trevor A. Thorpe, ed., Academic Press, New York, 1981; particularly the article by O.L. Gamborg and J.P. Shyluk at pp. 21-44, and the chart at page 33. The resulting mature plant, the chloroplasts of which contain integrated DNA of the vector of the invention, express the desired heterologous gene from the chloroplast DNA.

Vectors pFCC41::Tn5 #4 and pFCC71 in E. coli HB101 were deposited on June 10, 1986 in the American Type Culture Collection, Rockville, Maryland, and assigned ATCC Accession Numbers 67130 and 67129 respectively.

Claims

1. A plant having plastids in which the genomes of said plastids contain heterologous DNA.

2. The plant of claim 1, wherein said heterologous DNA includes DNA encoding a selectable marker protein.

3. The plant of claim 1, wherein said heterologous DNA includes a site for the insertion of a gene encoding a desired heterologous protein.

4. The plant of claim 3, wherein there is inserted, in said site, said gene encoding said desired heterologous protein.

5. The plant of claim 1, wherein said plastids comprise chloroplasts.

6. A vector capable of effecting the integration of a portion of itself into the genome of a plastid, said vector containing a transposon including a gene encoding a selectable marker protein and having insertion sequences on either side of said gene encoding said selectable marker portion, said vector further comprising, on either side of said insertion sequences, a first and second DNA region which together comprise a DNA region homologous with a DNA region of said genome of said plastid.

7. The vector of claim 6, wherein said transposon includes a site for the insertion of a gene encoding a desired heterologous protein.

8. The vector of claim 7, wherein there is inserted, at said site, said gene encoding said desired heterologous protein.

9. The vector of claim 8, wherein said plastid is a chloroplast.

10. The vector of claim 9, wherein said desired heterologous protein participates in a chemical reaction which occurs more efficiently inside said chloroplast than outside said chloroplast.

11. A plastid in which the genome thereof has integrated into it said integrating portion of the vector of claim 6.

12. The plastid of claim 11, wherein said plastid is a chloroplast.

13. A plant cell containing the plastid of claim 11.

14. A plant comprising a plurality of the cell of claim 13.

15. A method of producing a plant whose cells express a desired gene, comprising

   a) isolating plastids from a plant,

   b) transforming said plastids with the vector of claim 6,

   c) inserting said transformed plastids into plant cells, and

   d) regenerating a plant from said plant cells.

16. The method of claim 15, wherein said plastids comprise chloroplasts.

17. A plastid which is transformed with heterologous DNA.

18. The plastid of claim 17, wherein said plastid is a chloroplast.

pFCC41 :: Tn 5

HERBICIDE
RESISTANCE GENE

SELECTABLE
MARKER
(Kanamycin
Resistance)

BamHI

Tn 5

UNIQUE
SITE

CHLOROPLAST DNA

EcoRI        TS        TS        Bgl II

rbc L        atp B

EcoRI / Bam HI

pBR327
2,895 bp

FIG Ia

pFCC41 :: Tn5 #4

FIG 1b

FIG 2

SIZE OF INTERGENIC REGIONS IN

SPINACH 152 bp

PEA 152 bp

TOBACCO 146 bp

FIG 3

FIG 4

FIG 5

FIG 6

FIG 7

pRSNtS6

FIG 8

FIG 9

0 251 654

pEH 37B

FIG 10

pBR327 Δ Cla

EcoRI Cla I

pBR327 Δ Cla I

FIG II

CONSTRUCTION OF pFCC 66

pFCC 41 :: Tn5 #4          pEH 37B

LIGATE

pFCC 66

FIG 12

pFCC 66

FIG 13

FIG 14

FIG 15

pFCC73    CONSTRUCTION

FIG 16

pFCC59A

unique
Eco RI

p BR327 ,

Sal I

Sal I

Km
promoter

Bgl II

Km

Sal I

FIG 17

pFCC60A

EcoRI

Hind III

Bam HI

Tc

pSUP104

SalI

Km
promoter

Bgl II

Km

SalI

FIG 18

pFCC60B

EcoRI

Hind III

BamHI

Tc

pSUP104

SalI

Km

BglII

Km promoter

SalI

FIG 19

FIG 20

p EH 25K

FIG 21

pFCC6I

Eco RI

Hind III

Bam H I

Tc

pSUPI04

Sal I

Km

SD

Bgl II

FIG 22

pFCC62

FIG 23